# EUROPEAN PATENT SPECIFICATION

(11) **EP 3 406 197 B1**
(45) Date of publication and mention of the grant of the patent: **07.08.2019**
(21) Application number: 18173635.6
(22) Date of filing: 22.05.2018
(51) Int. Cl.: H01R 39/08, H04B 5/00, A61B 6/00, A61B 6/03

(54) **ROTARY JOINT HAVING A CAPACITIVE DATA LINK WITH MODULAR SUPPORT**
DREHVERBINDUNG MIT EINER KAPAZITIVEN DATENVERBINDUNG MIT MODULTRÄGER
JOINT ROTATIF AYANT UNE LIAISON DE DONNÉES CAPACITIVE AVEC SUPPORT MODULAIRE

(30) Priority: 22.05.2017 EP 17172301
(43) Date of publication of application: 28.11.2018
(73) Proprietor: Schleifring GmbH, 82256 Fürstenfeldbruck (DE)
(72) Inventor: KNOBL, Horst, 86956 Schongau (DE); WÖRL, Kathrin, 82281 Egenhofen (DE)
(74) Representative: Lohr, Jöstingmeier & Partner

(56) References cited:
- US-A1- 2008 279 302
- US-A1- 2016 127 052
- US-B2- 6 798 309

## Description

### Field of the invention

The invention relates to sliprings and contactless data links as may be used in Computed Tomography (CT) scanners.

### Description of the related art

In computed tomography (CT) scanners, usually large sliprings having a diameter of approximately 1 meter or more are required. The sliding tracks must be held at a supporting structure while insulating the sliding tracks from each other and from other machine parts. Furthermore, capacitive data links for coupling data with high data rates from a rotating part to a stationary part are provided.

US 5,054,189 discloses a slipring having a body of insulating material with pre-machined grooves into which a conductive sliding track is rolled.

US 5,734,218 discloses a drum-shaped slipring having a pre-formed insulating body with grooves into which sliding tracks are pressed. The body needs a rigid backing member as mechanical support.

A capacitive rotary joint for CT scanners is disclosed in US 5,600,697. A large diameter rotating ring carries a differentially driven strip coupler guiding a signal along this circumference of the ring. At the stationary side, there is a capacitive coupler picking up the signal from the near field of the strip coupler. The receiving coupler comprises two couplers held orthogonally to the transmission coupler. To obtain a sufficient coupling efficiency and therefore a sufficient signal level at the receiver, the coupler must be mounted in close proximity to the strip coupler.

A bidirectional capacitive coupler is disclosed in US 2013/0214614. Here, the channels for the communication from the rotating to the stationary side and vice versa are interleaved. The datalink transmitters are mounted on a comparatively large, solid and therefore expensive body of insulating material.

These embodiments have the disadvantage that they require comparatively solid insulating bodies which further require a significant amount of insulating material or additional rigid backing. Another disadvantage is the comparatively big size of the insulating body which makes handling and manufacturing of the insulating body difficult and expensive.

An arrangement for transmitting electrical signals and/or energy between parts that can be rotated in relation to each other is disclosed in US 6,798,309 B2.

### Summary of the invention

The problem to be solved by the invention is to provide a rotary joint component or slipring component having a capacitive data link in a comparatively lightweight and simple assembly.

Solutions of the problem are described in the independent claims. The dependent claims relate to further improvements of the invention.

A rotary joint component comprises a stationary part and a rotatable part. The rotatable part is rotatable against the stationary part. In a CT (computed tomography) scanner a gantry has a rotatable part which rotates around the patient. The rotatable part requires a power supply from the stationary part which is delivered by the rotary joint. The rotary joint further delivers data from the rotatable part to the stationary part. Therefore, the direction of a preferred data link is from the rotatable to the stationary part. In an alternate embodiment, there may be a data transfer from the stationary to the rotatable part, or in both directions. A data transfer in both directions may provide a data flow from an image detector of the rotatable gantry to a stationary part, and of control data from the stationary part to the rotatable part of the gantry.

In an embodiment, the rotatable part comprises a rotatable base for holding rotatable components, like at least one rotatable receiver coupler. The stationary part comprises a stationary base which holds at least one stationary transmitter coupler. The positions of the stationary transmitter coupler and the rotatable receiver coupler are adjusted such that the stationary transmitter coupler is capacitively coupled to the at least one rotatable receiver coupler, such that data may be transferred capacitively.

Herein, reference is simply made to a general coupler for capacitive coupling. It is preferred, if these couplers have pairs of couplers comprising a transmitter coupler and a receiver coupler. Most preferably, the transmitter coupler is a line or a strip line along the way of movement between the couplers. Specifically for rotatable movement this means that the transmitter coupler is arranged in a circular shape around the center axis of rotation. The receiver coupler may either by a line, a strip line or a simple capacitive coupling pad, which are preferably terminated with the characteristic impedance, to achieve a capacitive coupling to the transmitter coupler.

The at least one rotatable transmitter coupler and/or the at least one stationary transmitter coupler may be in a radial distance less than 10 mm, preferably less than 5 mm, and most preferably less than 3 mm from the facing at least one stationary receiver coupler and/or the at least one rotatable receiver coupler.

It is obvious that further components are required for a capacitive data transfer, like a data transmitter which preferably is at the rotatable part, and a data receiver which preferably is at the stationary part. These components are not part of the invention. The invention primarily relates to mechanical components and the basic coupling components for capacitive data coupling as well as mechanical slipring components.

The stationary transmitter coupler is mounted to or held by a stationary carrier. This stationary carrier comprises a circular-shaped sheet metal or plastic which is further held by the stationary base. The sheet may have a thickness between 0.2mm and 3mm. A metal sheet may have a thickness between 0.3mm and 1.5mm whereas a plastic sheet may have a thickness between 1.5mm and 3mm. A metal sheet material may comprise at least one of steel, stainless steel, brass, copper or any combination thereof. A plastic sheet material may comprise Polycarbonate, Polyethylene or any other suitable material with or without fiber reinforcement.

As the stationary carrier comprises sheet metal, it can be manufactured easily. This may for example be done by cutting a rectangular sheet of metal or plastic material having the right size, whereas the length corresponds to the circumference of the later arc-shape, and the width corresponds to the height. The sheet metal or plastic material gets its required stiffness and/or stability when it is mounted to the stationary base. To this sheet, at least one stationary transmitter coupler may be attached, for example by the use of a double-sided adhesive tape. As the sheet is a plane sheet, this may easily be done on a plane work bench. It is not necessary to handle a complex circular structure.

The stationary base may have means for holding the stationary carrier comprising sheet metal or plastic in a circular shape. Preferably, the stationary carrier may be fixed to the stationary base by screws, bolts or similar means. It may be glued, injected-molded or held otherwise to the stationary base. The stationary carrier may be press fitted into a groove in the stationary base, it may be bolted or screwed to the inner or outer diameter of a rotating base. Handling of two separate components, like the stationary carrier and the stationary base is comparatively simple, because both are flat components. As such, they may be shipped easily and assembled later at their final destination. This design also offers a larger flexibility. For example, different coupler configurations may be preassembled on stationary carriers and before finishing the slipring, one of these stationary carriers may be selected to assemble the desired configuration.

Making the stationary carrier of sheet metal also provides an electrical and/or electromagnetic shielding to the data link and specifically to the transmitter coupler. A shield may also be provided, if a plastic sheet material is used, which has a metallized surface. Alternatively an aluminium-plastic composite material may be cut as rectangular sheet and bent into a circular shape for use as carrier.

The at least one rotatable receiver coupler and/or the at least one stationary receiver coupler may be galvanically connected to its stationary carrier which further may be galvanically connected to its stationary base.

There may be at least one sliding track at the rotatable part. Such sliding tracks may be used for galvanic contact together with sliding brushes to provide a galvanic connection between the stationary and the rotatable part. Such a galvanic connection may be used for supplying electrical power from the stationary to the rotatable part.

At the stationary base, there may be a stationary electronic module support for supporting and holding a stationary electronic module. Such a stationary electronic module may provide connecting means, like connectors and/or amplifiers and/or transmitters and/or receivers, which may be connected to at least one stationary transmitter line and/or a stationary receiver line.

Furthermore, the stationary part may provide connecting means, like connectors and/or amplifiers and/or transmitters and/or receivers, which may be connected to at least one stationary transmitter line and/or a stationary receiver line. The rotatable electronic module and/or the stationary electronic module may also be connected to at least one of the sliding tracks. Such a connection may be used for grounding and/or for supplying power or other signals to the electronic modules.

### Description of Drawings

In the following the invention will be described by way of example, without limitation of the general inventive concept, on examples of embodiment with reference to the drawings.
- Figure 1: shows a first embodiment of the invention.
- Figure 2: shows the rotatable part without stationary part.
- Figure 3: shows the stationary part from the inner side.
- Figure 4: shows further details.
- Figure 5: shows a full view.
- Figure 6: shows a detail of a stationary carrier mounted to a stationary base

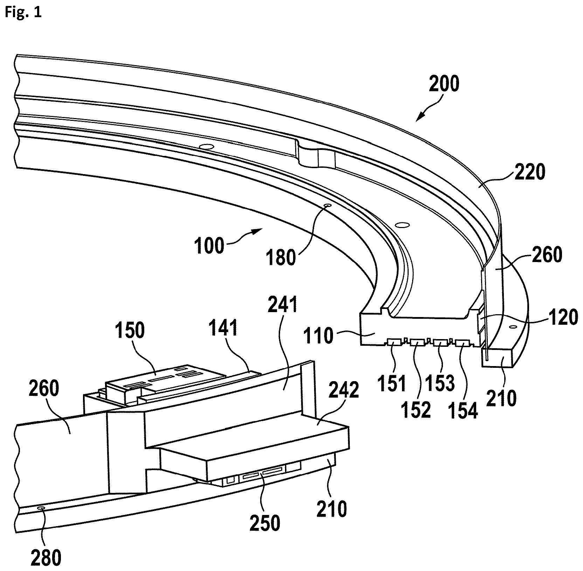

In Figure 1, a first embodiment of the invention is shown. It is obvious that the invention relates to a circular embodiment of the basic components. In this Figure and in some of the following Figures, a section is cut out of the ring-shaped parts to show their inner structure. A rotatable part 100 comprises a rotatable base 110 which may support a rotatable transmitter coupler 120 which may be a strip line. The rotatable base further may support a plurality of sliding tracks 151, 152, 153, and 154. There may be any other number of sliding tracks, as required. There may also be a rotatable electronic module 150 which may be mounted to a rotatable electronic module support 141. For mounting the rotatable part for example to a rotatable part of a CT scanner, there may be a plurality of rotatable screw holes 180.

This figure also shows a stationary part 200 which has a stationary base 210 holding a stationary carrier 260. On this stationary carrier 260, there is a stationary transmitter coupler 220 which may be a transmission line. There may also be a stationary electronic module 250 which may be supported by or mounted to a stationary electronic module support which may comprise a first support component 241 and a second support component 242. For mounting the stationary part for example to a stationary part of a CT scanner, there may be a plurality of stationary screw holes 280.

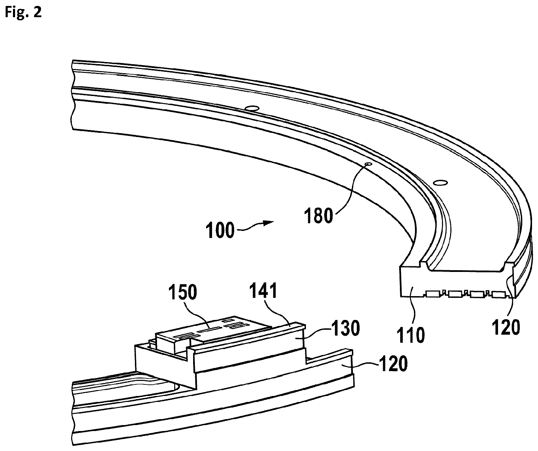

In Figure 2, the rotatable part 100 is shown without stationary part 200. Here, more details of the rotatable transmitter coupler 120, which may be mounted in a groove, are shown. Furthermore, the rotatable receiver coupler 130 is shown, which preferably is held by the rotatable electronic module support 141.

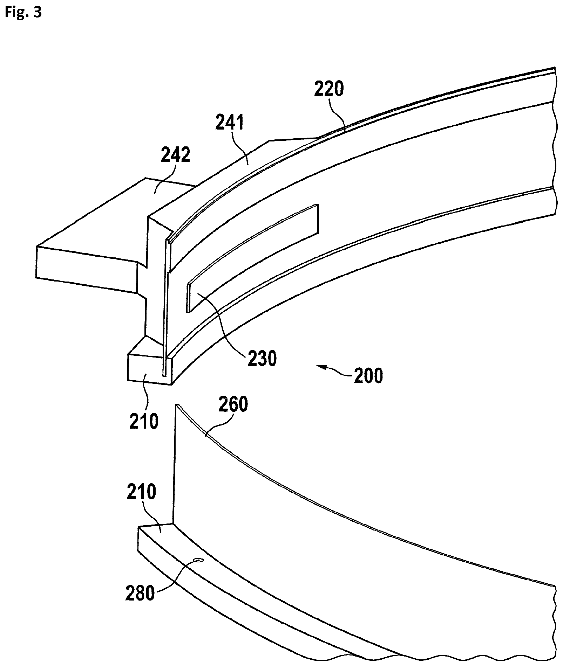

In Figure 3, the stationary part 200 is shown from the inner side. Here, the stationary receiver coupler 230 is shown, which may be held by the stationary electronic module support 241, 242. The width of the stationary carrier 260 depends on the number of data transmission links and typically is in the range of 20mm to 100mm.

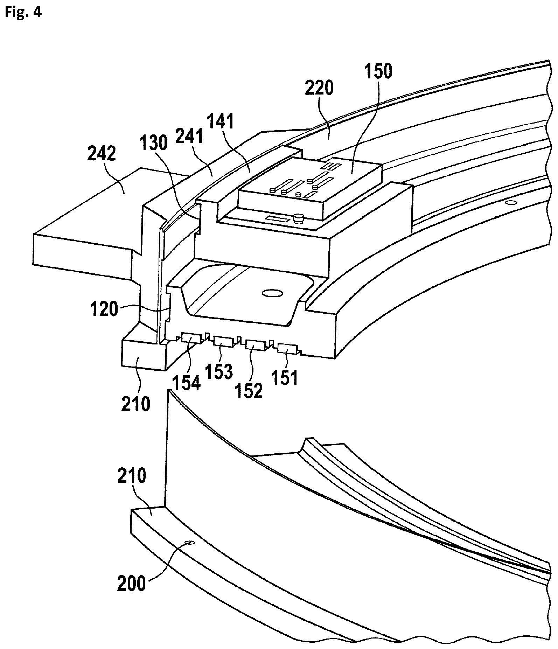

In Figure 4, further details are shown, specifically showing the interaction between the stationary transmitter coupler 220 and the rotatable receiver coupler 130 which basically runs on the same track, such that they are always in a close distance, which may be below 10 mm, more preferably below 5 mm, and most preferably below 3 mm.

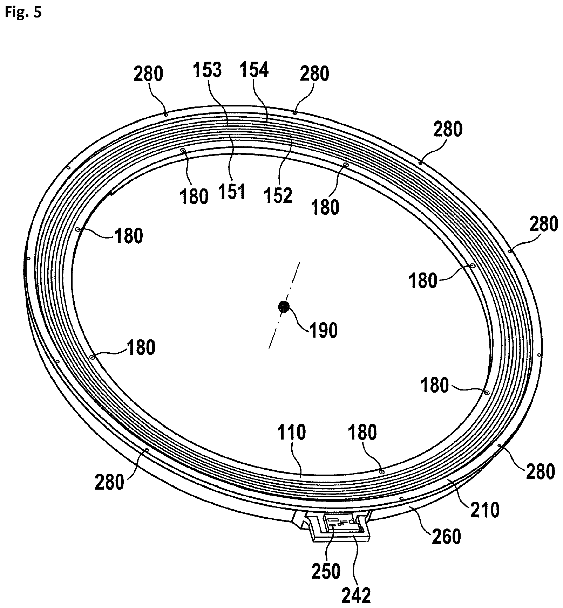

In Figure 5, a full view of the rotatable part 100 and the stationary part 200 is shown, with all associated components is shown. The axis of rotation 190 passes through the center at a right angle through a plane defined by the rotatable base 110.

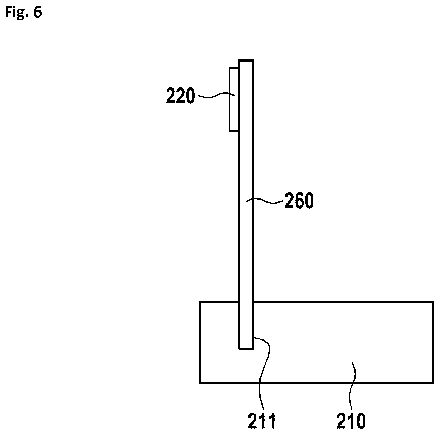

In Figure 6, details of the mounting of the stationary carrier into the stationary base are given. Preferably, the stationary base 210 has a slot 211 for holding the stationary carrier 260.

### List of reference numerals

- 100: rotatable part
- 110: rotatable base
- 120: rotatable transmitter coupler
- 130: rotatable receiver coupler
- 141: rotatable electronic module support
- 150: rotatable electronic module
- 151, 152, 153, 154: sliding tracks
- 180: rotatable screw hole
- 190: axis of rotation
- 200: stationary part
- 210: stationary base
- 211: slot
- 220: stationary transmitter coupler
- 230: stationary receiver coupler
- 241, 242: stationary electronic module support
- 250: stationary electronic module
- 260: stationary carrier
- 280: stationary screw hole

## Claims

1. Rotary joint component comprising a rotatable part (100) and a stationary part (200) stationary against the rotatable part (100),
the rotatable part (100) comprising a rotatable base (110) holding at least one rotatable receiver coupler (130),
the stationary part (200) comprising a stationary base (210) holding at least one stationary transmitter coupler (220), wherein the at least one stationary transmitter coupler (220) is capacitively coupled to the at least one rotatable receiver coupler (130),
**characterized in, that**
the stationary transmitter coupler (220) is mounted to a stationary carrier (260) which comprises circular shaped sheet metal which is further held by the stationary base (210), wherein
the stationary carrier (260) is held within a slot or groove (211) of the stationary base (210), and
the stationary carrier (260) comprises circular shaped sheet metal which has ends connected to each other.

2. Rotary joint component according to claim 1,
**characterized in, that**
the rotatable part (100) comprises at least one sliding track (151, 152, 153, 154) held by the rotatable base (110).

3. Rotary joint component according to any of the preceding claims,
**characterized in, that**
the rotatable part (100) holds at least one rotatable transmitter coupler (120) and the stationary part (200) holds at least one stationary receiver coupler (230) which is capacitively coupled to the at least one rotatable transmitter coupler (120).

4. Rotary joint component according to any of the preceding claims,
**characterized in, that**
the at least one rotatable transmitter coupler (120) and/or the at least one stationary transmitter coupler (220) are lines or striplines which are preferably terminated with their characteristic impedance.

5. Rotary joint component according to any of the preceding claims,
**characterized in, that**
the at least one rotatable receiver coupler (130) and/or the at least one stationary receiver coupler (230) are lines, striplines or pads which are preferably terminated with their characteristic impedance.

6. Rotary joint component according to any of the preceding claims,
**characterized in, that**
the at least one rotatable transmitter coupler (120) and/or the at least one stationary transmitter coupler (220) are in a distance less than 10mm, preferably 5mm and most preferably 3mm from the at least one rotatable receiver coupler (130) and/or the at least one stationary receiver coupler (230).

7. Rotary joint component according to any of the preceding claims,
**characterized in, that**
the at least one rotatable receiver coupler (130) and/or the at least one stationary receiver coupler (230) is galvanically connected to its stationary carrier which is galvanically connected to its stationary base.

8. Rotary joint component according to any of the preceding claims,
**characterized in, that**
the stationary carrier (260) comprises circular shaped sheet metal which has ends connected to each other by soldering or welding.

## Patentansprüche

1. Drehübertragerkomponente umfassend einen drehbaren Teil (100) und einen stationären Teil (200), der feststehend gegenüber dem drehbaren Teil (100) ist,
wobei der drehbare Teil (100) eine drehbare Basis (110) umfasst, die mindestens einen drehbaren Empfängerkoppler (130) hält,
wobei der stationäre Teil (200) eine stationäre Basis (210) umfasst, die mindestens einen stationären Senderkoppler (220) hält, wobei der mindestens eine stationäre Senderkoppler (220) kapazitiv mit dem mindestens einen drehbaren Empfängerkoppler (130) gekoppelt ist,
**dadurch gekennzeichnet, dass**
der stationäre Senderkoppler (220) an einen stationären Träger (260) angebracht ist, welcher ein kreisförmiges Blech umfasst, das weiterhin von der stationären Basis (210) gehalten wird, wobei
der stationäre Träger (260) in einem Schlitz oder einer Nut (211) der stationären Basis (210) gehalten ist, und
der stationäre Träger (260) ein kreisförmiges Blech umfasst, dessen Enden miteinander verbunden sind.

2. Drehübertragerkomponente nach Anspruch 1,
**dadurch gekennzeichnet, dass**
der drehbare Teil (100) mindestens eine Schleifbahn (151, 152, 153, 154) umfasst, die von der drehbaren Basis (110) gehalten wird.

3. Drehübertragerkomponente nach einem der vorhergehenden Ansprüche,
**dadurch gekennzeichnet, dass**
der drehbare Teil (100) mindestens einen drehbaren Senderkoppler (120) hält und der stationäre Teil (200) mindestens einen stationären Empfängerkoppler (230) hält, der kapazitiv mit dem mindestens einen drehbaren Senderkoppler (120) gekoppelt ist.

4. Drehübertragerkomponente nach einem der vorhergehenden Ansprüche,
**dadurch gekennzeichnet, dass**
der mindestens eine drehbare Sendekoppler (120) und/oder der mindestens eine stationäre Senderkoppler (220) Leitungen oder Streifenleitungen sind, die vorzugsweise mit ihrer charakteristischen Impedanz abgeschlossen sind.

5. Drehübertragerkomponente nach einem der vorhergehenden Ansprüche,
**dadurch gekennzeichnet, dass**
der mindestens eine drehbare Empfängerkoppler (130) und/oder der mindestens eine stationäre Empfängerkoppler (230) Leitungen, Streifenleitungen oder Pads sind, welche vorzugsweise mit ihrer charakteristischen Impedanz abgeschlossen sind.

6. Drehübertragerkomponente nach einem der vorhergehenden Ansprüche,
**dadurch gekennzeichnet, dass**
der mindestens eine drehbare Senderkoppler (120) und/oder der mindestens eine stationäre Senderkoppler (220) einen Abstand von weniger als 10 mm, bevorzugt 5 mm und am meisten bevorzugt 3 mm von dem mindestens einen drehbaren Empfängerkoppler (130) und/oder dem mindestens einen stationären Empfängerkoppler (230) haben.

7. Drehübertragerkomponente nach einem der vorhergehenden Ansprüche,
**dadurch gekennzeichnet, dass**
der mindestens eine drehbare Empfängerkoppler (130) und/oder der mindestens eine stationäre Empfängerkoppler (230) mit seinem stationären Träger, der galvanisch mit seiner stationären Basis verbunden ist, galvanisch verbunden ist.

8. Drehübertragerkomponente nach einem der vorhergehenden Ansprüche,
**dadurch gekennzeichnet, dass**
der stationäre Träger (260) ein kreisförmiges Blech aufweist, dessen Enden beispielsweise durch Löten oder Schweißen miteinander verbunden sind.

## Revendications

1. Composant d'articulation rotoïde comprenant une partie rotative (100) et une partie fixe (200) qui est fixe par rapport à la partie rotative (100),
la partie rotative (100) comprenant une base rotative (110) maintenant au moins un coupleur de récepteur rotatif (130),
la partie fixe (200) comprenant une base fixe (210) maintenant au moins un coupleur d'émetteur fixe (220), dans lequel l'au moins un coupleur d'émetteur fixe (220) est couplé capacitivement à l'au moins un coupleur de récepteur rotatif (130),
**caractérisé en ce que**
le coupleur d'émetteur fixe (220) est monté sur un support fixe (260) qui comprend une tôle en métal de forme circulaire qui est en outre maintenue par la base fixe (210), dans lequel
le support fixe (260) est maintenu au sein d'une fente ou rainure (211) de la base fixe (210), et
le support fixe (260) comprend une tôle en métal de forme circulaire qui a des extrémités connectées les unes aux autres.

2. Composant d'articulation rotoïde selon la revendication 1,
**caractérisé en ce que**
la partie rotative (100) comprend au moins une gorge de coulissement (151, 152, 153, 154) maintenue par la base rotative (110).

3. Composant d'articulation rotoïde selon l'une quelconque des revendications précédentes,
**caractérisé en ce que**
la partie rotative (100) maintient au moins un coupleur d'émetteur rotatif (120) et la partie fixe (200) maintient au moins un coupleur de récepteur fixe (230) qui est couplé capacitivement à l'au moins un coupleur d'émetteur rotatif (120).

4. Composant d'articulation rotoïde selon l'une quelconque des revendications précédentes,
**caractérisé en ce que**
l'au moins un coupleur d'émetteur rotatif (120) et/ou l'au moins un coupleur d'émetteur fixe (220) sont des lignes ou lignes à rubans qui sont de préférence fermées avec leur impédance caractéristique.

5. Composant d'articulation rotoïde selon l'une quelconque des revendications précédentes,
**caractérisé en ce que**
l'au moins un coupleur de récepteur rotatif (130) et/ou l'au moins un coupleur de récepteur fixe (230) sont des lignes, lignes à rubans ou plots qui sont de préférence fermés avec leur impédance caractéristique.

6. Composant d'articulation rotoïde selon l'une quelconque des revendications précédentes,
**caractérisé en ce que**
l'au moins un coupleur d'émetteur rotatif (120) et/ou l'au moins un coupleur d'émetteur fixe (220) sont à une distance inférieure à 10 mm, de préférence 5 mm et idéalement 3 mm de l'au moins un coupleur de récepteur rotatif (130) et/ou de l'au moins un coupleur de récepteur fixe (230).

7. Composant d'articulation rotoïde selon l'une quelconque des revendications précédentes,
**caractérisé en ce que**
l'au moins un coupleur de récepteur rotatif (130) et/ou l'au moins un coupleur de récepteur fixe (230) sont connectés galvaniquement à leur support fixe qui est connecté galvaniquement à sa base fixe.

8. Composant d'articulation rotoïde selon l'une quelconque des revendications précédentes,
**caractérisé en ce que**
le support fixe (260) comprend une tôle en métal de forme circulaire qui a des extrémités connectées les unes aux autres par brasage ou soudage.
